# FASCICULE DE BREVET EUROPEEN

(11) **EP 3 399 937 B1**
(45) Date de publication et mention de la délivrance du brevet: **31.01.2024**
(21) Numéro de dépôt: 17701172.3
(22) Date de dépôt: 05.01.2017
(51) Int. Cl.: A61B 34/30

(54) **MODULE ROBOTISABLE D'ENTRAÎNEMENT D'UN ORGANE MÉDICAL SOUPLE ALLONGÉ, ROBOT MÉDICAL ET SYSTÈME COMPRENANT UN TEL MODULE**
ROBOTISIERBARES MODUL ZUM ANTRIEB EINES LÄNGLICHEN FLEXIBLEN MEDIZINISCHEN ELEMENTS, MEDIZINISCHER ROBOTER UND SYSTEM MIT SOLCH EINEM MODUL
ROBOTIZABLE MODULE FOR DRIVING AN ELONGATED FLEXIBLE MEDICAL MEMBER, MEDICAL ROBOT AND SYSTEM INCLUDING SUCH A MODULE

(30) Priorité: 07.01.2016 FR 1650105
(43) Date de publication de la demande: 14.11.2018
(73) Titulaire: Robocath, 76000 Rouen (FR)
(72) Inventeur: DESTREBECQ, Fabien, 27520 Bourgtheroulde (FR); MAUREL, Julien, 60240 Montjavoult (FR); DEBOEUF, Sébastien, 76240 Bonsecours (FR); BENCTEUX, Philippe, 76160 Saint Martin du Vivier (FR)
(74) Mandataire: Plasseraud IP
(86) Numéro de dépôt international: PCT/FR2017/050028
(87) Numéro de publication internationale: WO 2017/118818

(56) Documents cités:
- EP-A1- 2 821 094
- EP-A2- 1 792 638
- EP-A2- 2 777 594
- US-A1- 2010 170 519
- US-A1- 2012 035 596
- US-A1- 2014 277 333

## Description

La présente invention est relative aux modules robotisables d'entraînement d'un organe médical souple allongé.

L'insertion manuelle d'un cathéter ou d'un guide dans un patient est un acte chirurgical relativement classique. Toutefois, cet acte étant monitoré sous rayons X, le chirurgien en charge de cet acte est soumis à une irradiation conséquente s'il réalise une telle opération sur de nombreux patients.

Afin de réduire les risques pour le chirurgien, on tente de robotiser une telle insertion. Cette robotisation est complexe, car la préhension du cathéter est difficile. Celui-ci est en effet glissant, et doit rester stérile. La fiabilité de ces systèmes robotisés malgré ces difficultés est un critère déterminant pour leur acceptation par la communauté médicale.

Récemment, il a été proposé dans US 7,927,310 un système d'entraînement gérant à la fois la translation et la rotation du cathéter. Le cathéter est maintenu sur une plaquette rotative par rapport à une base pour l'entraînement en rotation. La plaquette elle-même comporte un mécanisme d'entraînement en translation. De plus, on fait appel à des moteurs déportés, à demeure sur le bâti, et à des systèmes de transfert du mouvement jusqu'au cathéter. En effet, on préfère ne pas avoir les moteurs embarqués, pour des raisons d'alimentation en puissance, d'encombrement et de stérilité.

Bien que cette configuration apporte toute satisfaction, on cherche encore à en faciliter la mise en oeuvre par le personnel médical. Des critères déterminants sont la mise en service et la mise hors service rapides. La mise en service rapide, simple et instinctive, permet d'éviter au personnel une mauvaise mise en place du cathéter dans le robot, et par conséquent des problèmes ultérieurs. Une mise hors service rapide peut être nécessaire pour garantir une prise en charge manuelle de l'intervention par le personnel médical en cas de besoin.

Plus particulièrement, l'invention se rapporte à un module robotisable d'entraînement d'un organe médical souple allongé. Le module comprend une base.

Le module comprend un premier organe d'entraînement définissant un premier axe, et comprenant une première surface périphérique d'entraînement autour de ce premier axe, le premier organe d'entraînement étant monté mobile en rotation par rapport à la base autour du premier axe, et comprenant un organe de liaison à un moteur d'entrainement adapté pour entraîner le premier organe d'entrainement en rotation autour du premier axe.

Le module comprend un deuxième organe d'entraînement définissant un deuxième axe parallèle au premier axe, et comprenant une deuxième surface périphérique d'entraînement autour de ce deuxième axe, le deuxième organe d'entraînement étant monté mobile en rotation par rapport à la base autour du deuxième axe.

Le deuxième organe d'entraînement est également monté mobile par rapport au premier organe d'entraînement selon un degré de liberté autre qu'une rotation autour du deuxième axe, entre :
- une première configuration dans laquelle les première et deuxième surfaces périphériques d'entraînement se font face avec un premier espacement, et
- une deuxième configuration dans laquelle les première et deuxième surfaces périphériques d'entraînement se font face avec un deuxième espacement supérieur au premier espacement.

Le module comprend un système d'actionnement actionnable par un utilisateur adapté pour faire passer le deuxième organe d'entrainement au moins de l'une des première et deuxième configurations à l'autre des première et deuxième configurations.

US 2012/179,167 décrit un module robotisable présentant les caractéristiques ci-dessus.

Selon l'invention, le module comprend un système de transmission de mouvement pour transmettre le mouvement d'entrainement généré par le moteur d'entrainement au deuxième organe d'entrainement pour entraîner le deuxième organe d'entrainement en rotation autour du deuxième axe au moins dans toute configuration comprise entre les première et deuxième configurations.

Grâce à ces dispositions, on peut de manière très simple soit engager le cathéter avec le module robotisable, soit l'en désengager, en diminuant le risque de rendre le robot inopérant du fait de ces manoeuvres d'engagement/désengagement.

Dans des modes de réalisation préférés de l'invention, on peut éventuellement avoir recours en outre à l'une et/ou à l'autre des dispositions suivantes :
- le système de transmission de mouvement est opérant dans la configuration libre ;
- le système de transmission de mouvement comprend :
   _{·} un premier pignon coaxial avec le premier organe d'entrainement et formant un élément d'entrée du système de transmission de mouvement,
   . un pignon intermédiaire, présentant un axe de pignon intermédiaire parallèle au premier axe et décalé par rapport à celui-ci, le pignon intermédiaire étant en prise avec le premier pignon au moins dans toute configuration comprise entre les première et deuxième configurations,
   . une transmission entre le pignon intermédiaire et le deuxième organe d'entraînement, transmettant le
   mouvement de rotation du pignon intermédiaire autour de l'axe de pignon intermédiaire en ledit mouvement de rotation du deuxième organe d'entrainement autour du deuxième axe.
- le module comprend un système élastique sollicitant le deuxième organe d'entraînement de sa deuxième configuration vers sa première configuration,
   Et le système d'actionnement est actionnable pour faire passer le deuxième organe d'entrainement de la première configuration et à la deuxième configuration en comprimant ledit système élastique ;
- le système élastique sollicite le système d'actionnement solidaire du deuxième organe d'entrainement ;
- le module comprend un système de verrouillage adapté pour alternativement verrouiller le deuxième organe d'entrainement dans sa configuration libre ou l'en libérer, le système d'actionnement étant adapté pour commander le système de verrouillage ;
- le système d'actionnement est actionnable électriquement par l'utilisateur ;
- au moins un organe d'entraînement est également monté mobile par rapport à la base selon un mouvement de translation selon son axe ;
- ledit au moins un organe d'entraînement est monté mobile par rapport à la base selon un mouvement de translation selon son axe sur une course de translation,
   Et le premier organe d'entraînement comporte une jupe déformable, frottant sur la base lors de la rotation du premier organe d'entraînement par rapport à la base, et définissant un pourtour fermé sur la base sur l'intégralité de la course de translation ;
- le module robotisable comprend en outre un capot solidaire de la base et définissant avec celle-ci un boîtier définissant un espace intérieur dans lequel sont disposés au moins une partie du premier organe d'entraînement, au moins une partie du deuxième organe d'entraînement et au moins une partie du système d'actionnement, et dans lequel une portion d'actionnement du système d'actionnement, une partie du premier organe d'entraînement et une partie du deuxième organe d'entraînement dépassent hors du boîtier.

Selon un autre aspect, l'invention se rapporte à un robot médical en kit comprenant une partie à demeure et une partie amovible, la partie à demeure comprenant un moteur et un premier accouplement, la partie amovible comprenant un tel module robotisable muni d'un deuxième accouplement complémentaire du premier accouplement,
les premier et deuxième accouplements comprennent au moins une surface de came adaptée pour faire tourner l'un par rapport à l'autre le premier et le deuxième accouplements par rapport à une direction d'assemblage lors d'un assemblage de la partie amovible à la partie à demeure selon la direction d'assemblage.

Dans un mode de réalisation préféré de l'invention, on peut éventuellement avoir recours à la disposition suivante : le premier accouplement comprend une pluralité de saillies de forme concave, et le deuxième accouplement comprend une pluralité complémentaire de logements de forme complémentaire.

Dans un mode de réalisation préféré de l'invention, le premier accouplement comprend un cône de centrage, une saillie mobile par rapport au cône de centrage selon une direction de coulissement, et un organe de sollicitation sollicitant la saillie par rapport au cône de centrage lors d'un assemblage de la partie amovible à la partie à demeure.

Selon un autre aspect, l'invention se rapporte à un système médical comprenant un organe médical souple creux allongé selon un axe d'élongation, et un tel robot médical ou un tel module robotisable, l'organe médical souple creux allongé étant pris entre les première et deuxième surfaces périphériques d'entraînement dans la première configuration, le premier organe d'entraînement étant entraînable en rotation par rapport à la base autour du premier axe pour générer une translation de l'organe médical souple allongé selon son axe d'élongation.

Dans un mode de réalisation préféré de l'invention, on peut éventuellement avoir recours à la disposition suivante : le premier organe d'entraînement est entraînable en translation par rapport à la base selon le premier axe pour générer une rotation de l'organe médical souple allongé autour de son axe d'élongation.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1a est une vue schématique de côté d'une installation d'artériographie robotisée.
- la figure 1b est une vue de dessus d'une partie de la figure 1a,
- les figures 2a-2c sont des schémas illustratifs des modes de déplacement des organes à entraîner,
- la figure 3 est une vue en perspective d'un exemple de réalisation d'un module robotisable,
- la figure 3a est une vue en perspective d'un détail de réalisation de la figure 3,
- la figure 4a est une vue de dessus du détail de la figure 3, dans une première configuration, le capot ayant été enlevé,
- la figure 4b est une vue similaire à la figure 4a dans une autre configuration,
- la figure 5 est une vue de côté du mécanisme de la figure 4a, illustré sans le boîtier,
- les figures 6a et 6b sont deux vues selon des perspectives différentes en éclaté d'un même accouplement,
- la figure 7 est une vue en perspective en éclaté d'un deuxième mode de réalisation,
- la figure 8 est une vue en coupe verticale de l'accouplement du module au moteur,
- la figure 9a est une vue en coupe d'un détail de la figure 8 dans une première configuration,
- la figure 9b est une vue correspondant à la figure 9a dans une deuxième configuration,
- les figures 10a et 10b sont des vues en perspective en éclaté correspondant aux figures 6a, 6b pour un deuxième exemple d'un accouplement,
- les figures 11a et 11b sont des vues schématiques du module d'entraînement selon un mode de réalisation dans deux configurations différentes,
- les figures 12a, 12b et 13a, 13b sont des vues correspondant aux figures 11a, 11b pour d'autres modes de réalisation,
- la figure 14 est une vue en perspective de la partie montée sur le robot d'un accouplement selon un troisième exemple, et
- la figure 15 est une vue en éclaté du dispositif de la figure 14.

Sur les différentes figures, les mêmes références désignent des éléments identiques ou similaires.

L'invention est définie par la revendication indépendante 1. D'autres modes de réalisation sont définis par les revendications dépendantes 2 à 1.

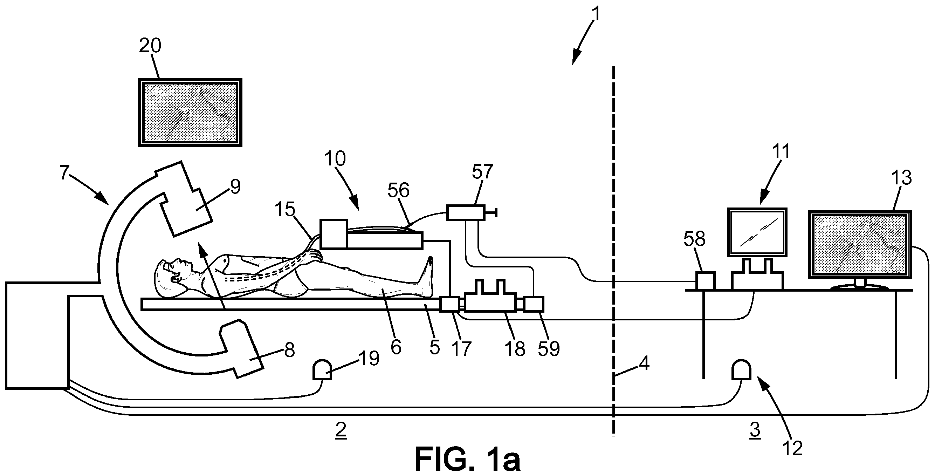

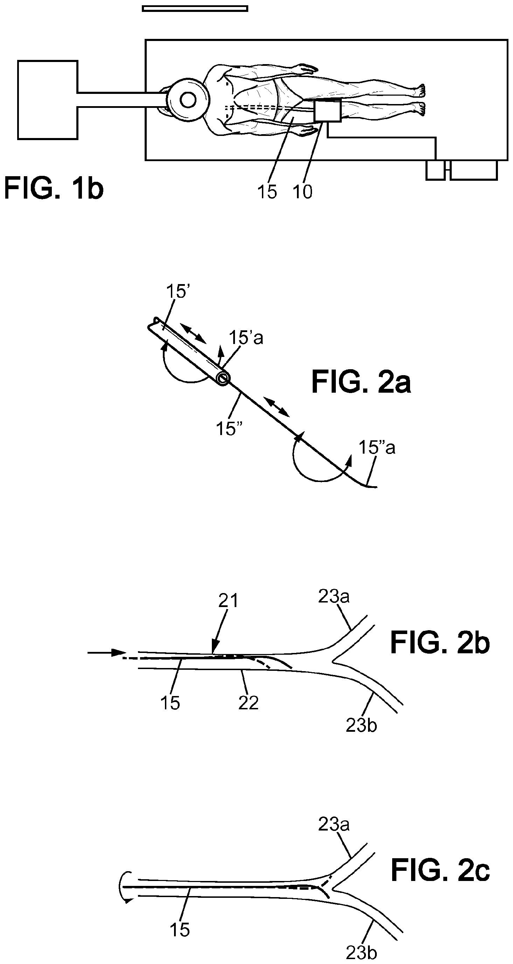

La figure 1a représente schématiquement une installation d'artériographie 1. L'installation d'artériographie 1 est divisée en deux endroits distincts, une salle d'opérations 2 et une salle de commande 3. La salle de commande 3 peut être distante, ou proche de la salle d'opération 2, séparée de celle-ci par une simple paroi 4, par exemple un écran, mobile et/ou amovible, opaque aux rayons X. Les matériels de la salle d'opération 2 et de la salle de commande 3 sont reliés entre eux de manière fonctionnelle, par voie filaire, sans fil ou réseau, ....

La salle d'opérations 2 comprend une table d'opérations 5 recevant un patient 6. La salle d'opérations 2 peut également comprendre un imageur médical 7, notamment un imageur par rayons X, comprenant une source 8 et un détecteur 9 disposés de part et d'autre du patient, éventuellement mobiles par rapport au patient.

L'installation d'artériographie 1 comprend un robot 10 disposé dans la salle d'opérations 2.

L'installation d'artériographie 1 comprend un poste de commande 11 disposé dans la salle de commande 3. Le poste de commande 11 est adapté pour commander à distance le robot 10. L'installation d'artériographie 1 peut également comprendre, disposée dans la salle de commande 3, une ou plusieurs commandes distantes 12 de l'imageur 7, communiquant avec l'imageur 7 pour commander celui-ci à distance. L'installation d'artériographie 1 peut également comprendre, disposé dans la salle de commande 3, un écran 13, communiquant avec l'imageur 7, pour visualiser en temps réel dans la salle de commande 3 les images acquises par l'imageur 7.

Le robot 10 peut déplacer un organe médical souple allongé 15 à introduire dans le corps d'un patient. A titre d'organe médical souple allongé 15, il peut par exemple s'agir d'un organe à introduire dans un canal d'un patient, et à déplacer dans ce canal, notamment une artère ou une veine d'un patient, à travers un désilet ménageant une ouverture d'accès dans le patient. L'organe médical souple allongé peut être notamment un cathéter. En variante, l'organe médical souple allongé peut être un guide pour cathéter. Un guide est généralement de diamètre transversal inférieur à celui du cathéter, qui est généralement creux sur une portion proche du patient, voire sur la totalité de sa longueur, de sorte que le guide puisse se déplacer à l'intérieur de celui-ci, notamment à l'intérieur du corps du patient. Le guide peut également comporter une extrémité recourbée, comme il sera décrit plus en détail ci-après.

Le robot 10 est commandable à partir du poste de commande 11 pour entraîner l'organe médical souple allongé par rapport au patient selon au moins un degré de liberté, comme ce sera décrit en détails par la suite. Le robot peut comprendre un boîtier de communication 17 servant à l'interfaçage avec le poste de commande 11. Au besoin, le robot 10 peut comprendre un boîtier de commande 18 en local, destiné à commander le robot depuis la salle d'opérations 2 si nécessaire.

On notera d'ailleurs que toutes les commandes et les retours disponibles dans la salle de commande 3 peuvent être également disponibles dans la salle d'opérations 2 en vue d'une opération en local, comme par exemple une commande 19 de l'imageur et un écran 20 permettant de visualiser les images acquises par l'imageur 7.

L'organe médical souple allongé 15 creux peut être raccordé à un raccord 56 permettant l'injection d'un produit de contraste facilitant l'imagerie à l'intérieur du patient. L'installation d'artériographie peut comprendre un injecteur 57 de produit de contraste raccordé au raccord 56, commandable par une commande 58 disposée dans la salle de commande 3. Une commande 59 de l'injecteur de produit de contraste peut également être présente en local dans la salle d'opérations 2.

Dans ce qui suit, on utilisera la référence 15 pour désigner alternativement le guide 15", le cathéter 15', ou de manière générale un organe médical souple allongé à introduire dans le corps d'un patient. Il peut par exemple s'agir d'un cathéter interventionnel. Un tel cathéter interventionnel peut être de diamètre inférieur à un cathéter extérieur, de manière à être guidé à l'intérieur de celui-ci, coaxialement à l'intérieur du patient, et être creux de manière à être guidé sur le guide à l'intérieur du patient.

Le raccord 56 comprend une branche principale 75 à travers laquelle passent juxtaposés le cathéter 15' et le guide 15''. L'extrémité distale de la banche principale 75 est assemblée à un cathéter extérieur (non représenté), s'étendant à l'intérieur du patient et à l'intérieur duquel s'étendent le cathéter 15' et le guide 15". Le produit de contraste est injecté à l'intérieur du cathéter extérieur par l'intermédiaire d'une branche secondaire 76 du raccord 56.

La figure 2a représente les divers degrés de liberté envisageables avec le présent système. On visualise le guide 15" avec son extrémité avant 15"a légèrement courbée par rapport à l'axe longitudinal principal du guide, et débouchant par l'extrémité avant 15'a du cathéter 15'. Le cathéter 15' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le cathéter 15' peut être soumis à un mouvement combiné des deux mouvements simples décrits ci-dessus.

Le cas échéant, le cathéter 15' peut être soumis à deux mouvements combinés des deux mouvements simples décrits ci-dessus, selon des combinaisons différentes.

Le guide 15" peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le guide 15" peut être soumis à un mouvement combiné des deux mouvements simples décrits ci-dessus.

Le cas échéant, le guide 15" peut être soumis à deux mouvements combinés des deux mouvements simples décrits ci-dessus, selon des combinaisons différentes.

Dans certains cas, le cathéter est lui-même pourvu d'une extrémité courbe, soit pour permettre la navigation sur le même principe qu'un guide, soit pour faciliter le positionnement dans une zone anatomique présentant une courbure particulière.

Sur la figure 2b, on a représenté une artère 21 d'un patient comprenant un tronc principal 22 et deux branches 23a, 23b débouchant sur le tronc principal. La figure 2b illustre le déplacement d'un organe médical souple allongé 15 (ici un guide 15") selon une translation entre une position reculée représentée en pointillés et une position avancée représentée en traits pleins. Sur la figure 2c, dans la même artère, on a représenté une rotation de l'organe médical souple allongé 15 entre une première position, représenté en pointillés, où l'organe médical souple allongé est prêt à être soumis à une translation en direction de la branche 23a, et une deuxième position, représentée en traits pleins, où l'organe médical souple allongé est prêt à être soumis à une translation en direction de la branche 23b.

L'assemblage du robot et du cathéter et/ou du guide est appelé un « système médical ».

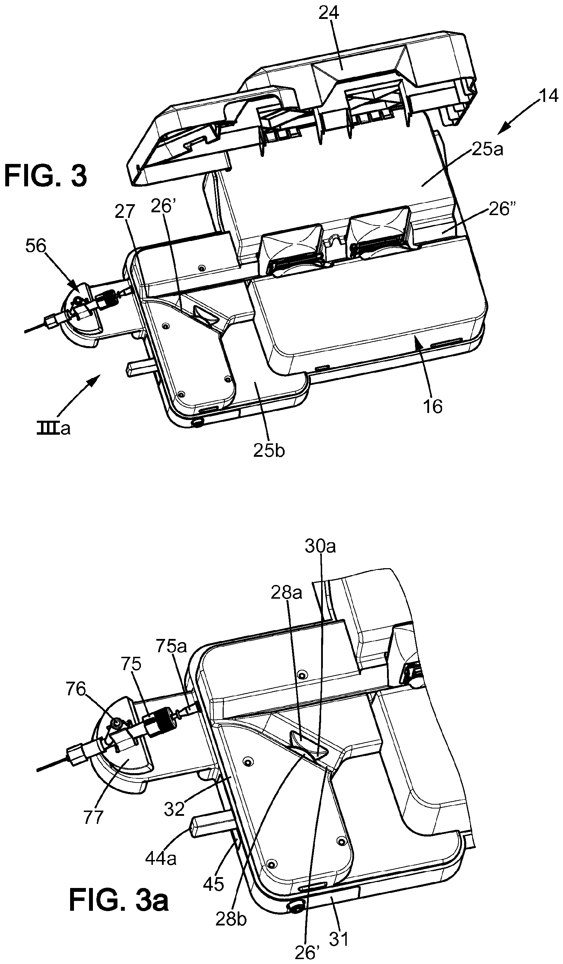

La figure 3 représente une vue en perspective d'un module d'entraînement 14. Dans cet exemple de réalisation, le module d'entrainement 14 est réalisé à usage unique, et est prévu pour être assemblé de manière stérile sur un système motorisant. Le module d'entraînement 14 comporte un boîtier 16 et un couvercle 24. Le couvercle 24 est mobile par rapport au boîtier 16 entre deux configurations, respectivement ouverte et fermée. La configuration représentée est la configuration ouverte. Dans cette configuration, le cathéter 15' et le guide 15" sont accessibles. Dans la configuration fermée, le cathéter 15' et le guide 15" ne sont pas accessibles au niveau du module 14.

Dans l'exemple présenté, le module d'entraînement 14 entraîne le cathéter 15' et le guide 15". Toutefois, ceci est illustratif, et l'invention pourrait être mise en oeuvre dans un système entraînant uniquement le cathéter 15' ou uniquement le guide 15".

Dans le présent exemple, le module d'entraînement 14 comprend une première portion 25a entraînant le guide 15', et une deuxième portion 25b entraînant le cathéter 15". La première portion est sensiblement telle que décrit dans QT FR2015/051566. On rappellera que ce système permet de commander la translation et/ou la rotation du guide par une succession de mouvements infinitésimaux répétés générés par une paire de doigts d'actionnement. Pour différentes raisons (vitesse, sécurité, fiabilité), on peut utiliser deux paires de doigts, comme par exemple dans le présent mode de réalisation, par exemple en décalage de phase.

Le guide 15" s'étend dans un canal 26". Le cathéter 15" s'étend dans un canal 26'. Les canaux 26' et 26" se rejoignent en un canal commun 27 dans lequel s'étendent à la fois le cathéter 15' et le guide 15". On a par exemple recours à un cathéter dit « à échange rapide », c'est-à-dire présentant une ouverture d'accès au guide dans sa paroi latérale. L'ouverture d'accès en question se situe en aval du canal commun 27. Ceci permet au guide 15" de courir en parallèle du cathéter 15' et à l'extérieur de celui-ci au moins jusqu'à l'ouverture d'accès, où le guide 15" entre à l'intérieur du cathéter jusqu'à dépasser de l'extrémité distale du cathéter à l'intérieur du corps du patient comme représenté sur la figure 2a.

On remarquera que, sur l'illustration, le raccord 56 est porté par un support 77 mobile, qui est présenté dans une configuration escamotée facilitant l'installation du cathéter 15' et du guide 15" dans leurs canaux 26', 26" respectifs. Suite à cette installation, le support 77 est déplacé, rabattu, de sorte à ce que l'extrémité 75a de la branche principale soit en regard du canal commun 27. Ceci permet la bonne insertion du cathéter 15' et du guide 15" à travers le raccord 56.

La deuxième portion 25b sera décrite plus en détail ci-après, notamment en relation avec la figure 3a.

Comme on peut le voir en particulier sur la figure 3a, on observe un premier organe d'entraînement 28a définissant un premier axe 29a, et comprenant une première surface périphérique d'entraînement 30a autour de ce premier axe 29a.

Le premier organe d'entraînement 28a est monté mobile en rotation par rapport au boîtier 16 autour du premier axe 29a (dans le cas présent, vertical).

Un deuxième organe d'entraînement 28b définit un deuxième axe 29b, et comprend une deuxième surface périphérique d'entraînement 30b autour de ce deuxième axe 29b.

Le deuxième organe d'entraînement 28b est monté mobile en rotation par rapport au boîtier 16 autour du deuxième axe 29b.

Le deuxième axe 29b est parallèle au premier axe 29a. Il est également espacé par rapport à celui-ci, en configuration d'entraînement, qui est la configuration représentée sur la figure 3a, de sorte qu'une partie de la première surface périphérique d'entraînement 30a et une partie de la deuxième surface périphérique d'entraînement 30b sont en regard l'une de l'autre, espacées d'un espace de l'ordre de l'épaisseur du cathéter 15'. Ainsi, la partie de la première surface périphérique d'entraînement 30a et la partie de la deuxième surface périphérique d'entraînement 30b en question font saillie dans le canal 26' .

Le boîtier 16 comporte une base 31 et un capot 32 assemblés ensemble, comme visible sur la figure 3a. La base 31 et le capot 32 assemblés ensemble définissent ensemble un volume intérieur 41 dans lequel est disposé le mécanisme. Seule une partie des organes d'entraînement 28a, 28b, dépasse hors du volume intérieur, afin de permettre l'entraînement du cathéter. La majeure partie du mécanisme est disposée dans le volume intérieur, afin de réduire le risque d'accéder par accident au mécanisme.

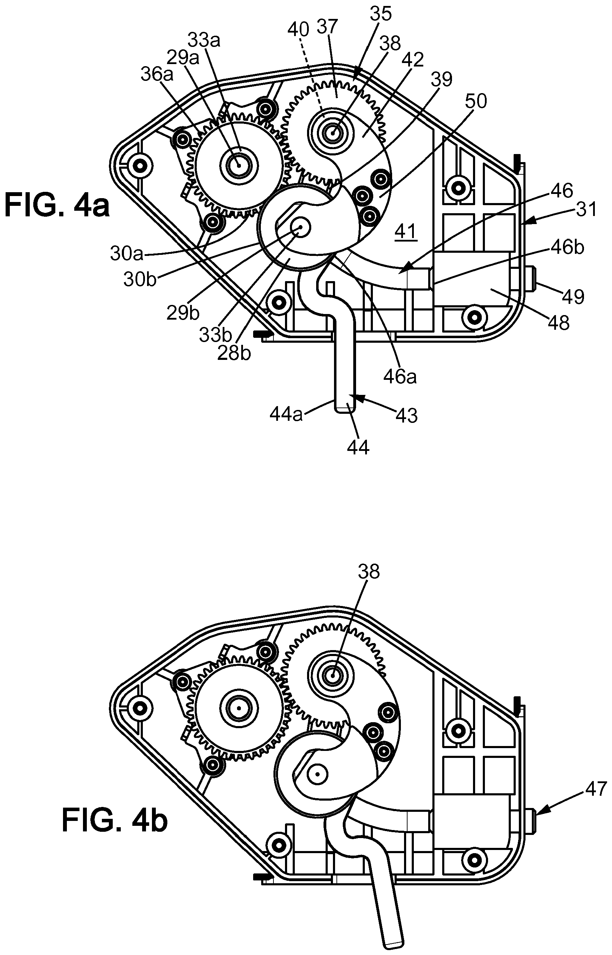

Le deuxième organe d'entraînement 28b est monté mobile par rapport au premier organe d'entraînement 28a selon un degré de liberté autre qu'une rotation autour du deuxième axe 29b, entre :
- une première configuration, dite configuration d'entraînement (figure 4a) dans laquelle les première et deuxième surfaces périphériques d'entraînement 30a, 30b se font face avec un premier espacement, et
- une deuxième configuration, dite configuration libre (figure 4b) dans laquelle les première et deuxième surfaces périphériques d'entraînement 30a, 30b se font face avec un deuxième espacement supérieur au premier espacement.

Au cours de ce déplacement, le deuxième organe d'entraînement 28b prend une infinité de configurations intermédiaires entre la première et la deuxième configuration. De plus, il est possible que la configuration libre ne soit pas la configuration ultime du système, dans le sens de déplacement allant de la configuration d'entraînement à la configuration libre, et qu'une poursuite du mouvement du deuxième organe d'entraînement 28b dans ce sens au-delà de cette configuration soit possible. De même, il est possible que la configuration d'entraînement ne soit pas la configuration ultime du système, dans le sens de déplacement allant de la configuration libre à la configuration d'entraînement, et qu'une poursuite du mouvement du deuxième organe d'entraînement 28b dans ce sens au-delàa de cette configuration soit possible. La configuration d'entrainement est définie par un serrage donné d'un cathéter 15' de diamètre donné.

La description ci-dessous présente un exemple de mécanisme permettant le passage de l'une à l'autre de ces configurations.

Le premier organe d'entraînement 28a est fixé à un arbre 33a, ayant pour axe l'axe d'entrainement 29a, et entraîné par un moteur 34. De cette manière, l'actionnement du moteur 34 génère la rotation du premier organe d'entraînement 28a autour de l'axe 29a. L'arbre 33a permet ainsi de faire une liaison entre le moteur 34 et le premier organe d'entraînement 28a.

Le mécanisme comprend également un système de transmission de mouvement 35 transmettant le mouvement d'entrainement généré par le moteur d'entrainement 34 au deuxième organe d'entrainement 28b. Il s'agit d'entrainer le deuxième organe d'entrainement 28b en rotation autour du deuxième axe 29b dans le bon sens, c'est-à-dire dans le sens opposé au sens de rotation du première organe d'entraînement 28a, afin que les deux organes d'entraînement 28a et 28b entraînent en translation le cathéter 15'.

Dans l'exemple présenté, le système de transmission de mouvement 35 comprend un premier pignon 36a coaxial avec le premier organe d'entrainement 28a. Le premier pignon 36a forme un élément d'entrée du système de transmission de mouvement 35.

Le système de transmission de mouvement 35 comprend un pignon intermédiaire 37, présentant un axe 38 de pignon intermédiaire parallèle au premier axe 29a et décalé par rapport à celui-ci. Le pignon intermédiaire 37 est en prise avec le premier pignon 36a à la fois dans les configurations d'entrainement (figure 4a) et libre (figure 4b) .

Le système de transmission de mouvement 35 comprend une transmission 39 entre le pignon intermédiaire 37 et le deuxième organe d'entraînement 28b, transmettant le mouvement de rotation du pignon intermédiaire 37 autour de l'axe 38 de pignon intermédiaire en le mouvement de rotation du deuxième organe d'entrainement 28b autour du deuxième axe 29b.

Dans le présent exemple, la transmission 39 comprend une courroie qui est solidaire en rotation du pignon intermédiaire 37 autour de l'axe 38 et du deuxième organe d'entraînement 28b autour de l'axe 29b.

Ainsi, le pignon intermédiaire 37 est fixé sur un arbre intermédiaire 40 dont l'axe est l'axe 38. L'arbre intermédiaire 40 est solidaire de la courroie.

Le deuxième organe d'entraînement 28b est solidaire d'un arbre 33b d'axe le deuxième axe 29b. L'arbre 33b est solidaire de la courroie.

L'arbre 33b est supporté par un étrier 42, qui est monté libre en rotation à la fois sur l'arbre 40 et sur le deuxième arbre 33b.

Ainsi, le deuxième organe d'entrainement 28b peut passer de sa configuration d'entraînement, représentée sur la figure 4a, à sa configuration libre, représentée sur la figure 4b, par rotation autour de l'axe 38.

On remarquera que, dans la configuration libre de la figure 4b, le système de transmission de mouvement 35 reste opérant. En d'autres termes, le moteur 34 entraîne en rotation le deuxième organe d'entraînement 28b également dans cette configuration. Ceci n'est d'ailleurs pas vrai uniquement dans la configuration libre, mais dans toute configuration intermédiaire entre la configuration d'entrainement et la configuration libre, voire au-delà. Le système de transmission de mouvement 35 étant toujours opérant, ceci permet d'assurer que, lorsque le deuxième organe d'entrainement 28b passe de sa configuration libre à sa configuration d'entraînement, le cathéter est entraîné sans problème par les deux organes d'entraînement.

Grâce à ces dispositions, on peut également garantir un entraînement de cathéters de différents diamètres à l'aide du même mécanisme, et/ou appliquer des efforts de serrage différents sur un cathéter donné (par rapprochement des deux organes d'entrainement 28a, 28b l'un de l'autre).

Bien que l'exemple ci-dessus fasse intervenir un système de transmission de mouvement 35 particulier, il s'agit d'un exemple illustratif, particulièrement compact, d'autres variantes de réalisation semblant envisageables pour parvenir à la même cinématique.

Le degré de liberté pour passer de la configuration d'entraînement libre à la configuration d'entraînement est une rotation autour d'un axe parallèle aux axes des organes d'entraînement. Toutefois, il s'agit d'un exemple de réalisation, d'autres mises en oeuvre apparaissant possibles.

Le mécanisme comprend un système d'actionnement 43 actionnable par un utilisateur. Le système d'actionnement 43 fait passer, quand actionné, le deuxième organe d'entrainement 28b de sa configuration d'entraînement à sa configuration libre.

Le système d'actionnement 43 comprend un levier 44 lié à l'étrier 42, par exemple en une zone de fixation 50. Le levier 44 comprend une extrémité d'actionnement 44a dépassant hors du boîtier 16 par une fente 45 allongée (figure 3a). Le déplacement de l'extrémité d'actionnement 44a du levier 44 dans la fente 45 allongée entre une première et une deuxième position permet le placement du deuxième organe d'entrainement 28b de sa configuration d'entraînement à sa configuration libre.

Le cas échéant, un système élastique 46, tel qu'un ressort, sollicite le levier 44 vers sa première position. Notamment, ce faisant, il sollicite le deuxième organe d'entrainement 28b vers sa configuration d'entraînement.

Ainsi, lorsque le deuxième organe d'entraînement 28b est passé de sa position d'entraînement à sa position libre par action de l'utilisateur sur l'actionneur 43, cela comprime le système élastique 46.

Le système élastique 46 comprend par exemple un ressort, dont la première extrémité 46a est fixée à l'actionneur 43, et la deuxième extrémité 46b à la base 31.

Par ailleurs, la position de la deuxième extrémité 46b par rapport à la base peut être réglable par un mécanisme de réglage 47. Un tel mécanisme permet de modifier la force de serrage des organes d'entraînement 28a, 28b sur un cathéter 15' donné, et/ou de s'adapter à différents diamètres de cathéters.

Le mécanisme de réglage 47 comprend par exemple un écrou 48 solidaire de la base 31, dans lequel est vissé une vis 49. La deuxième extrémité 46b du ressort est en appui sur une surface de butée de la vis 49. Le vissage de la vis 49 dans l'écrou 48 permet de modifier la longueur de l'espace dans lequel le ressort peut s'étendre.

L'étanchéité du boîtier est garantie par le joint 81 solidaire du premier organe d'entraînement 28a, et frottant sur la base 31. Il s'agit d'une étanchéité dynamique. Le contact du joint 81 sur la base 31 entoure une ouverture de la base 31 à travers laquelle le boîtier 16 est couplé à l'étage de motorisation 51.

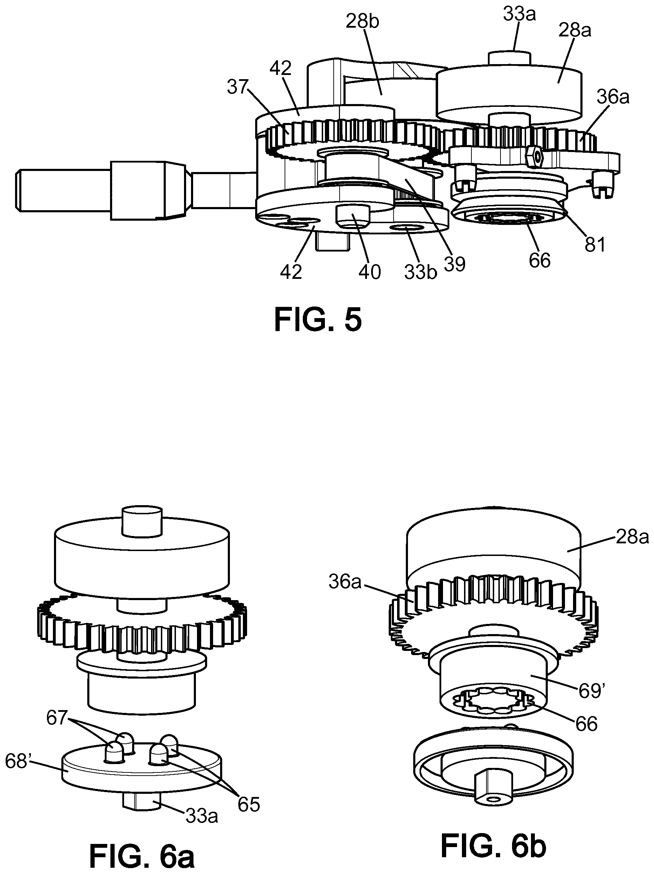

Les figures 6a et 6b présentent un exemple de réalisation d'un accouplement entre l'étage de motorisation 51 et le boîtier 16 (le joint 81 n'est pas représenté sur cette figure). Dans cet exemple, l'arbre 33a entraîné par le moteur comporte un organe d'accouplement 68' qui présente une pluralité de tétons 65 (dans le cas présent, 4 tétons) identiques. Les tétons 65 sont répartis, par exemple équi-répartis, selon un cercle passant par l'axe 29a. L'organe d'entraînement 28a est solidaire d'un organe d'accouplement 69' comportant une pluralité de logements 66 répartis selon le cercle passant par l'axe 29a. Les logements 66 sont identiques et sont de forme complémentaire aux tétons 65. Les logements 66 sont disposés tangents les uns avec les autres de manière à former une couronne, de sorte que, quelle que soit la position relative des tétons 65 et des logements 66 autour de l'axe 29a, les tétons 65 sont toujours tous au moins partiellement en face d'un logement 66 respectif.

Les tétons 65 peuvent présenter une extrémité bombée 67 pour guider l'accouplement du boitier sur l'arbre, moyennant si nécessaire une légère rotation de l'organe d'entrainement 28a autour de l'axe 29a d'un décalage au plus égal à une demie denture.

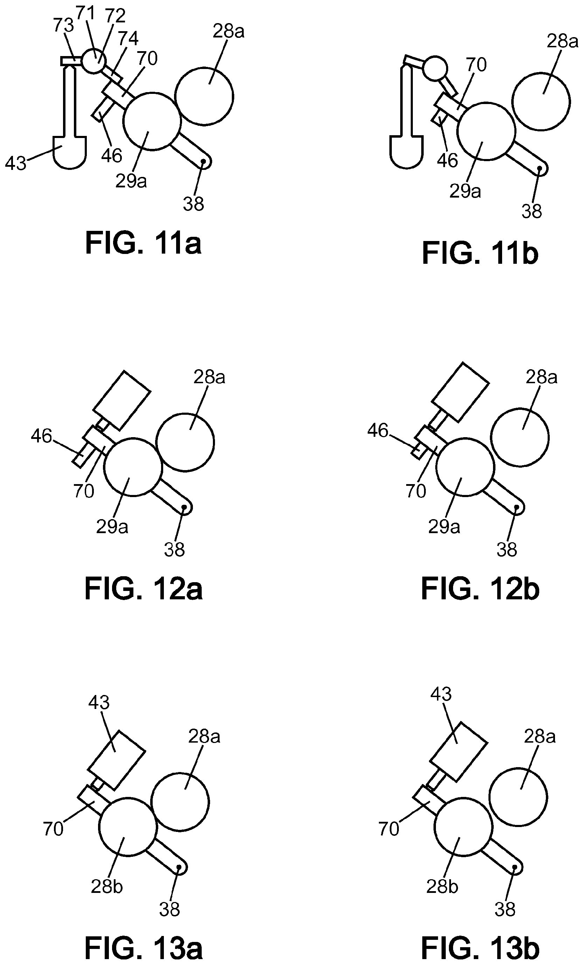

La figure 11a représente une variante de réalisation du système d'actionnement décrit ci-dessus en relation avec les figures 4a et 4b. Plus précisément, la figure 11a représente le module en configuration d'entraînement, alors que la figure 11b le représente en configuration libre. Comme on peut le voir sur ces figures :
- L'organe d'entraînement 28a est fixe dans le boîtier lors du passage entre les configurations d'entraînement et libre,
- L'organe d'entraînement 29a est monté sur un support 70 (comme par exemple l'étrier 50), qui est lui-même monté rotatif dans le boîtier autour d'un axe intermédiaire 38 lors du passage entre les configurations d'entraînement et libre.

La description ci-dessus est également valable pour la variante de réalisation des figures 12a et 12b et celle des figures 13a et 13b.

Sur la figure 11a, on utilise une bascule 71 pour transmettre le mouvement de l'actionneur 43 à l'organe d'entraînement 29a. La bascule 71 est montée pivotante autour d'un axe 72, par exemple parallèle à l'axe 38. La bascule 71 comporte un premier bras 73 en contact avec l'actionneur 43, et un deuxième bras 74 en contact avec le support 70.

L'actionneur 43 cause la rotation de la bascule 71, dont le deuxième bras 74 appuie sur le support 70, ce qui cause la rotation du support 70 autour de son axe 38 (figure 11b).

Ce mouvement compresse un ressort 46.

Selon une première variante, l'arrêt de l'actionnement par l'utilisateur de l'actionneur 43 ramène automatiquement le système en configuration d'entraînement par détente du ressort 46.

En variante, on peut prévoir un système de verrouillage dans la configuration libre (figure 11b). On peut par exemple mettre en oeuvre un système du type « push-pull » (« pousser-tirer ») comme pour l'insertion de cartes dans des lecteurs de carte. En configuration libre, un actionnement de l'actionneur 43 par l'utilisateur déverrouille le système de verrouillage, de sorte que le système est ramené en configuration d'entraînement par détente du ressort 46.

Dans les exemples ci-dessus, on peut avoir recours un actionnement mécanique de l'actionneur par contact par un utilisateur. Une telle réalisation permet de garantir l'actionnement par un utilisateur même en cas de panne électrique.

En variante, comme représenté schématiquement sur la figure 12a, on peut avoir recours à un actionneur commandé électriquement. Dans ce cas, par sécurité, la figure 12b représente le système au repos (c'est-à-dire sans application de courant électrique). En cas d'application d'un courant, l'actionneur 43 entraîne le support 70 en rotation autour de l'axe 38 par rapport à la position de repos, tendant ainsi le ressort 46. En cas de coupure d'alimentation électrique, par exemple pour commander le passage en configuration libre, le ressort 46 tire le support 70 comme représenté sur la figure 12b. Egalement, en cas de coupure de courant accidentelle, le cathéter peut être dégagé du mécanisme.

On peut avoir recours à tout type d'actionneur linéaire. L'étanchéité au niveau de la commande est assurée par l'intermédiaire d'un connecteur électrique.

Comme discuté ci-dessus, on peut avoir recours soit à une commande de libération à actionner en continu qui, en cas d'arrêt de la commande, ramène automatiquement le système en configuration d'entraînement. En variante, on peut prévoir de verrouiller le système en configuration libre.

On notera d'ailleurs que l'actionneur 43 peut agir directement sur le support 70, plutôt que par l'intermédiaire d'une bascule.

Comme représenté sur les figures 13a, 13b, en variante, on n'a pas nécessairement recours à un ressort 46. Par exemple, le support 70 est directement lié à l'actionneur, de sorte à suivre les mouvements de l'actionneur.

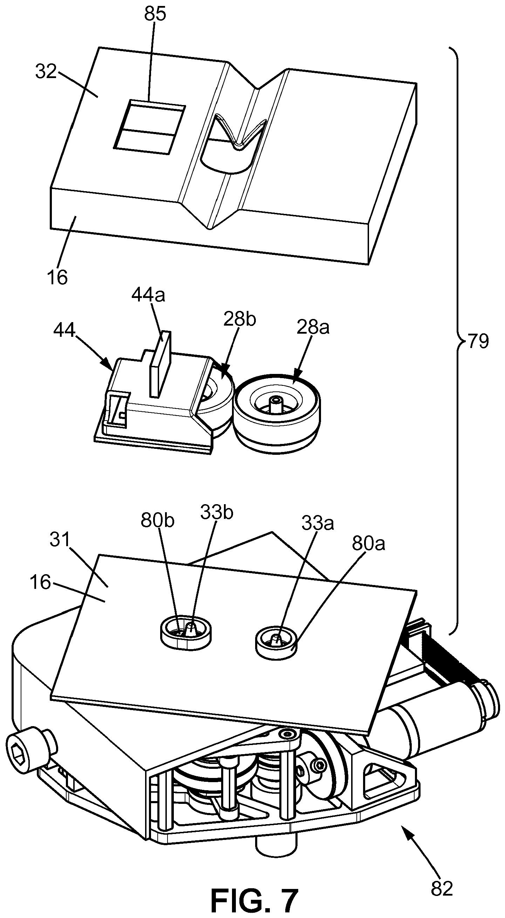

La figure 7 illustre ci-dessous un deuxième mode de réalisation. Le deuxième mode de réalisation diffère du premier mode de réalisation par un certain nombre de caractéristiques. Une première différence est que la partie consommable 79 à usage unique comprend le boîtier 16 (le capot 32 et la base 31) logeant les organes d'entrainement 28a et 28b, et l'actionneur 44.

La base 31 comprend une première ouverture 80a d'où dépasse le premier arbre 33a et une deuxième ouverture 80b d'où dépasse le deuxième arbre 33b. La deuxième ouverture 80b est grande, de manière à autoriser une course du deuxième arbre 33b par rapport à la base 31 (correspondant au passage du deuxième organe d'entrainement 29b entre deux configurations).

Ce mode de réalisation nécessite une liaison stérile entre le premier organe d'entrainement 28a et la base 31, de manière à réduire le risque de contamination du cathéter par le mécanisme et/ou de grippage du mécanisme par des substances convoyées par le cathéter.

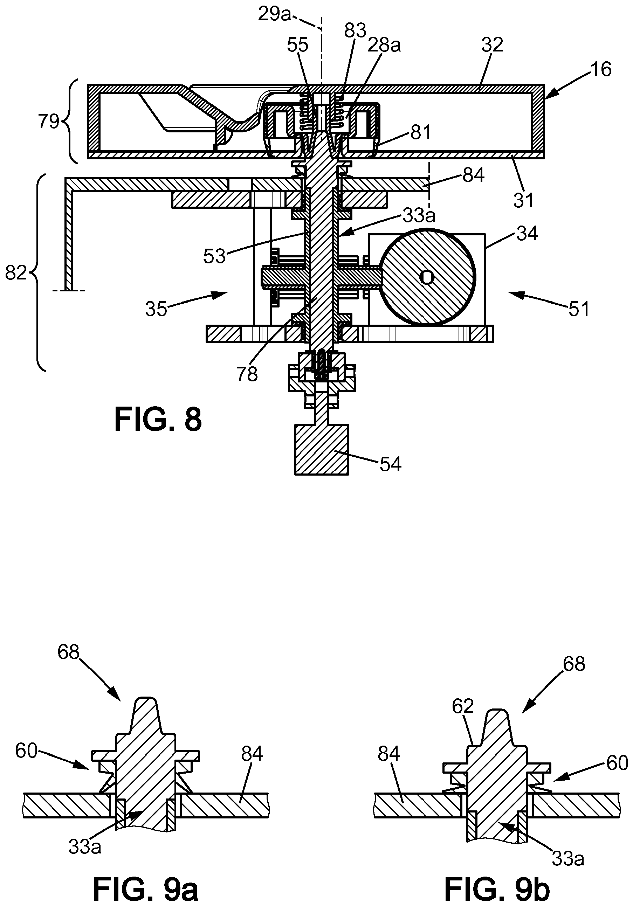

Selon un mode de réalisation, et comme visible sur la figure 8, l'organe d'entrainement 28a est solidaire d'une jupe 81 déformable, frottant sur la base 31, et définissant un pourtour fermé sur la base 31.

La figure 8 illustre en coupe verticale la motorisation de l'organe d'entraînement 28a par le moteur 34. Le module d'entraînement 14 comprend le boîtier 16, ainsi qu'un étage de motorisation 51.

La figure 8 représente ainsi un robot médical comprenant une partie à demeure 82 et une partie amovible, la partie à demeure 82 comprenant un moteur 34 et un premier accouplement 68, la partie consommable 79, amovible, étant munie d'un deuxième accouplement 69 complémentaire du premier accouplement 68.

On comprendra que le cas échéant, le robot médical représenté assemblé sur la figure 8 pourra être disponible en kit, avec d'une part la partie à demeure et, d'autre part, la partie amovible à lui assembler. La partie amovible, réalisée consommable, pourra être disponible en grandes quantités.

L'arbre 33a est en prise avec le premier organe d'entraînement 28a par un accouplement qui sera présenté en détail plus loin.

Dans le cas présent, on illustre également sur la figure 8 un mode de réalisation dans lequel le module entraîne en rotation le cathéter 15' autour de son axe d'élongation. Cet entraînement en rotation se fait par un mouvement de translation de l'organe d'entrainement 28a selon son axe 29a. Dans le cas présent, le cathéter 15' étant serré entre les organes d'entrainement 28a, 28b, un déplacement de l'un des organes d'entrainement par rapport à l'autre selon cet axe génère un roulement du cathéter 15', et donc une rotation de celui-ci autour de son axe d'élongation.

Dans le cas présent, la rotation est une rotation limitée à moins d'un tour par rapport à une position de départ. On peut prévoir que la position neutre de départ soit une position intermédiaire, permettant ainsi une rotation du cathéter dans un sens et dans l'autre, selon le sens de translation de l'organe d'entrainement 28a.

Dans l'exemple présenté, l'arbre 33a est réalisé en deux parties présentant des formes complémentaires permettant une rotation solidaire des deux parties autour de l'axe 29a. Une première partie est une âme interne 78 solidaire de l'organe d'entrainement 28a, et une deuxième partie est une enveloppe externe 53 coopérant avec le moteur 34. Par ailleurs, l'âme interne 78 est libre de coulisser par rapport à l'enveloppe externe 53 selon l'axe 29a. Un actionneur 54 commande le déplacement de l'âme interne 78 selon l'axe 29a. Un moyen élastique 83 tel qu'un ressort de rappel renvoie le premier organe d'entrainement 28a vers une position de repos selon l'axe 29a.

La commande de l'actionneur 54 permet de déplacer l'organe d'entrainement 28a selon l'axe 29a par l'intermédiaire de l'âme interne 78, l'arbre 33a restant en toute position en coopération avec le moteur 34.

L'actionnement du moteur 34 permet d'entraîner en rotation l'organe d'entrainement 28a comme décrit ci-dessus.

La jupe 81 est suffisamment longue et déformable pour garantir la stérilité à l'interface entre l'organe d'entrainement 28a et la base 31 selon l'intégralité de la course de l'organe d'entrainement 28a selon l'axe 29a.

Comme on le comprend de la description ci-dessus, dans ce mode de réalisation où la partie consommable comprend un nombre réduit de composants, le système de transmission de mouvement 35 est réalisé à l'intérieur du boîtier 84 de l'étage de motorisation 51.

On peut prévoir deux exemples de réalisation pour intégrer le pignon 36a. Selon une première variante, le pignon 36a est solidaire en translation de l'arbre 33a. Dans ce cas, le pignon intermédiaire 37 est d'épaisseur suffisante pour toujours engrainer avec le pignon 36a, quelle que soit sa position selon l'axe 29a. En variante, l'arbre 33a est solidaire en rotation, mais libre en translation par rapport au pignon 36a. Pour des raisons d'encombrement, la première variante pourra être mise en oeuvre dans le mode de réalisation des figures 4a et 4b, et la deuxième variante pourra être mise en oeuvre dans le mode de réalisation de la figure 8.

Les figures 9a et 9b présentent un détail de réalisation quant à la réalisation de l'étanchéité du robot. On rappellera qu'il s'agit ici d'une étanchéité dynamique, l'arbre 33a tournant pour entraîner en translation le cathéter.

L'arbre 33a, et notamment l'âme 78, est solidaire d'un joint 60. On choisit un joint 60 suffisamment déformable pour que, dans la position extrême haute, représentée sur la figure 9a, il soit frottant sur le boîtier 84 et, dans la position extrême basse, représentée sur la figure 9b, il soit déformé de manière à appuyer sur le boîtier 84. Cette réalisation est rendue possible par la faible course de rotation du cathéter 15' (plage de rotation inférieure à +/- 180°). En effet, dans le cas d'un cathéter dit « à échange rapide », on souhaite éviter de grandes courses de rotation qui pourraient causer un enroulement du guide à l'extérieur du cathéter.

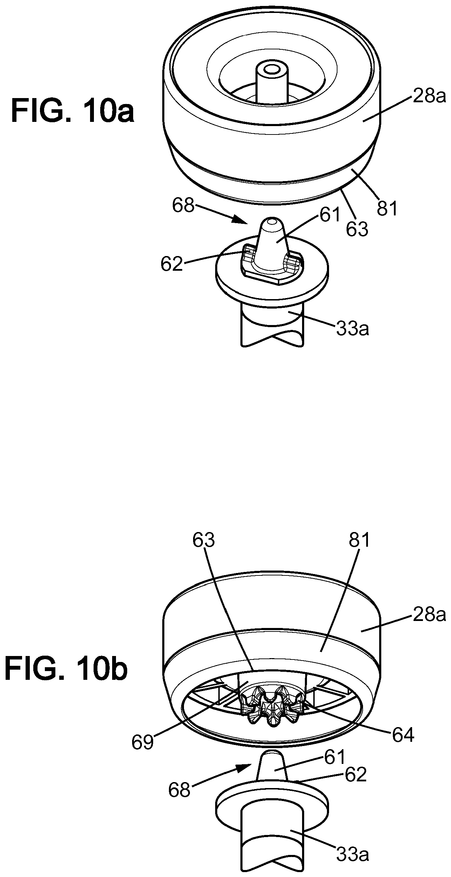

Les figures 10a et 10b représentent en perspective un accouplement du boîtier 16 sur l'étage de motorisation 51. Pour simplifier, le boîtier 16 n'est pas représenté sur cette figure.

Comme visible sur la figure 7, l'arbre 33a présente un organe d'accouplement 68 muni d'un cône de centrage 61 et une (ou plusieurs) dent d'engrènement 62. L'organe d'entrainement 28a comprend un organe d'accouplement 69, complémentaire de l'organe d'accouplement 68. Notamment, l'organe d'accouplement 69 comprend une cavité 63, complémentaire du cône de centrage 61, et une pluralité de dents d'entraînement 64, par exemple réparties sur la totalité du pourtour périphérique. Lors de l'assemblage du boîtier 16 à l'étage de motorisation 51 selon une direction d'assemblage (sensiblement la direction de l'axe 29a), le cône de centrage 61 coopère avec la cavité 63 pour guider l'accouplement, jusqu'à ce que la dent 62 coopère avec une des dents 64 de l'organe d'entrainement 28a, moyennant si nécessaire une légère rotation par effet de came de l'organe d'entrainement 28a autour de l'axe 29a d'un décalage au plus égal à une demie denture.

Comme représenté sur la figure 7, l'extrémité d'actionnement 44a n'est pas nécessairement disposée au niveau de la tranche du boîtier 16, mais peut être par exemple en surface supérieure. Dans ce mode de réalisation, le capot 32 comporte en surface supérieure une fenêtre 85 à travers laquelle projette l'extrémité d'actionnement 44a de l'actionneur 44 qui est relié au deuxième organe d'entraînement 28b. L'actionneur 44 comprend par exemple un capot profilé entourant partiellement le deuxième organe d'entraînement 28b, et lié à celui-ci de manière à ce qu'ils puissent tous deux se déplacer vers la configuration libre (l'arbre 33b se déplace alors dans l'ouverture 80b), tout en autorisant la rotation du deuxième organe d'entraînement 28b autour de son axe.

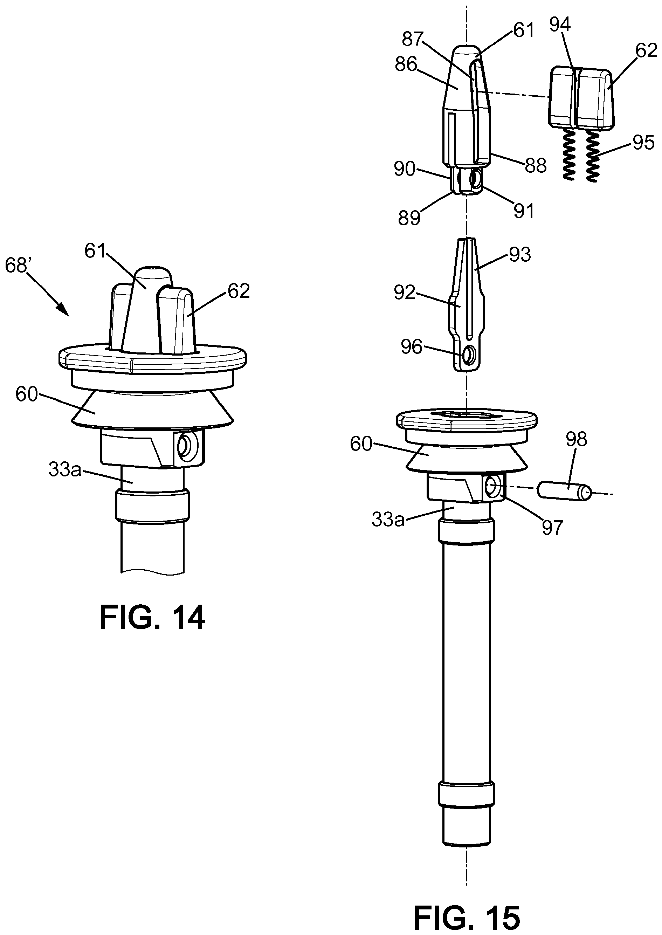

Selon un autre exemple de réalisation, un accouplement peut comprendre un organe d'accouplement 68 côté robot, tel que représenté sur la figure 14, complémentaire d'un organe d'accouplement côté consommable (non représenté à nouveau). L'organe d'accouplement côté consommable est par exemple du type de l'organe d'accouplement 69 illustré ci-dessus en référence à la figure 10b. Selon une particularité de l'organe d'accouplement 68, le cône de centrage 61 et la dent 62 sont mobiles l'une par rapport à l'autre. Notamment, ils sont montés en translation l'un par rapport à l'autre, notamment selon la direction d'assemblage du consommable sur le robot.

Pour celà, on peut par exemple envisager que le cône de centrage 61 comporte une enveloppe externe 86 comportant une extrémité cônique offrant la fonction de centrage du cône de centrage 61. L'enveloppe externe 86 présente également un logement interne 87 accessible par une ouverture latérale 88 et une ouverture inférieure 89. L'extrémité inférieure 90 de l'enveloppe externe 86 définit également un pallier 91 pour un axe transversal qui sera décrit plus loin.

Le cône de centrage 61 comprend également une âme interne 92 susceptible d'être placée dans le logement interne 87 depuis le bas par l'ouverture inférieure 89. L'âme interne 92 comporte une fente 93 allongée selon la direction de translation. Cette fente débouche du côté de l'extrémité supérieure de l'âme interne 92. L'âme interne 92 comporte également un pallier 96.

La dent 62 comporte une forme 94 complémentaire de la fente 93. Il s'agit par exemple d'un renfoncement creusé sur les deux faces principales opposées de la dent.

Un organe de sollicitation 95 est monté entre la dent 62 et l'enveloppe externe 86. Celui-ci sollicite la dent 62 selon la direction de translation vers le haut par rapport à l'enveloppe 86. Par exemple, on utilise deux ressorts, à titre d'organe de sollicitation. Ces deux ressorts sont alors disposés de part et d'autre de la forme 94. L'organe de sollicitation 95 est par exemple fixé à la dent 62. Par exemple, la dent 62 comporte, au niveau de sa face inférieure, un alésage recevant une partie d'extrémité du ressort.

Le système qui vient d'être décrit est monté comme suit. L'organe de sollicitation 95 est comprimé, et la dent 62 les portant est insérée à travers le logement interne 87 par l'ouverture latérale 88 jusqu'à ce que la forme 94 se situe dans le logement interne 87. Les ailes de la dent dépassent alors de part et d'autre de l'enveloppe externe 86.

L'organe de sollicitation 95 est relâché et prend appui sur l'enveloppe externe 86 (sur une face interne du logement interne 87), et sollicite la dent 62 vers le haut (position de la figure 14).

L'âme interne 92 est montée à travers l'ouverture inférieure 89, la fente 93 coopérant avec la forme 94. Les paliers 91 et 96 sont alors alignés.

L'ensemble est montée sur la base de l'axe 33a, les paliers 91 et 96 venant en alignement d'un logement 97 de celle-ci. Un axe 98 est inséré à travers le logement 97 et les paliers 91 et 96 pour fixer l'organe d'accouplement 68 sur la base de l'axe 33a.

En fonctionnement, l'organe d'accouplement 68 est solidaire en rotation de la base de l'axe 33a par l'intermédiaire de l'axe 98. Lors de l'accouplement, si la dent 62 ne fait pas face à un creux complémentaire de l'organe d'accouplement complémentaire, mais à une surface en saillie de celui-ci, cette surface en saillie déplace la dent 62 vers le bas par rapport à l'enveloppe extérieure 86 en comprimant l'organe de sollicitation 95. Lors d'une rotation ultérieure de l'organe d'accouplement 68, lorsque la dent 62 se retrouve face à un creux complémentaire de l'organe d'accouplement complémentaire, l'organe de sollicitation 95 pousse la dent 62 en prise (position de la figure 14) avec celui-ci.

Ce mode de réalisation permet d'utiliser une géométrie fortement crénelée des organes d'accouplement, qui permet de transmettre, en utilisation, un couple important.

Ainsi, selon un autre aspect indépendant du premier, il semble qu'une invention se rapporte à un module robotisable d'entraînement d'un organe médical souple allongé comprenant :
- une base 31,
- un premier organe d'entraînement 28a définissant un premier axe 29a, et comprenant une première surface périphérique d'entraînement 30a autour de ce premier axe 29a, le premier organe d'entraînement 28a étant monté mobile en rotation par rapport à la base 31 autour du premier axe 29a, et comprenant un organe de liaison 33a à un moteur d'entrainement 34 adapté pour entraîner le premier organe d'entrainement 28a en rotation autour du premier axe 29a,

Dans lequel le premier organe d'entraînement 28a est également monté mobile par rapport à la base 31 selon un mouvement de translation selon son axe 29a sur une course de translation,
Dans lequel le premier organe d'entraînement 28a comporte une jupe 81 déformable, frottant sur la base 31 lors de la rotation du premier organe d'entraînement par rapport à la base, et définissant un pourtour fermé sur la base 31 sur l'intégralité de la course de translation.

## Revendications

1. Module robotisable d'entraînement d'un organe médical souple allongé comprenant :
- une base (31),
- un premier organe d'entraînement (28a) définissant un premier axe (29a), et comprenant une première surface périphérique d'entraînement (30a) autour de ce premier axe (29a), le premier organe d'entraînement (28a) étant monté mobile en rotation par rapport à la base (31) autour du premier axe (29a), et comprenant un organe de liaison (33a) à un moteur d'entraînement (34) adapté pour entraîner le premier organe d'entraînement (28a) en rotation autour du premier axe (29a),
- un deuxième organe d'entraînement (28b) définissant un deuxième axe (29b) parallèle au premier axe (29a), et comprenant une deuxième surface périphérique d'entraînement (30b) autour de ce deuxième axe (29b), le deuxième organe d'entraînement (29b) étant monté mobile en rotation par rapport à la base (31) autour du deuxième axe (29b),
le deuxième organe d'entraînement (28b) étant également monté mobile par rapport au premier organe d'entraînement (28a) selon un degré de liberté autre qu'une rotation autour du deuxième axe (29b), entre :
- une première configuration dans laquelle les première et deuxième surfaces périphériques d'entraînement (30a, 30b) se font face avec un premier espacement, et
- une deuxième configuration dans laquelle les première et deuxième surfaces périphériques d'entraînement (30a, 30b) se font face avec un deuxième espacement supérieur au premier espacement,
- un système d'actionnement (43) actionnable par un utilisateur adapté pour faire passer le deuxième organe d'entraînement (28b) au moins de l'une des première et deuxième configurations à l'autre des première et deuxième configurations,
**Caractérisé par** un système de transmission de mouvement (35) pour transmettre le mouvement d'entrainement généré par le moteur d'entrainement (34) au deuxième organe d'entrainement (28b) pour entraîner le deuxième organe d'entraînement (28b) en rotation autour du deuxième axe (29b) au moins dans toute configuration comprise entre les première et deuxième configurations
Et comprenant un système élastique (46) sollicitant le deuxième organe d'entraînement (28b) de sa deuxième configuration vers sa première configuration,
Et dans lequel le système d'actionnement (43) est actionnable pour faire passer le deuxième organe d'entraînement (28b) de la première configuration à la deuxième configuration en comprimant ledit système élastique (46) .

2. Module robotisable selon la revendication 1, dans lequel le système de transmission de mouvement (35) est opérant dans la configuration libre qui est la deuxième configuration.

3. Module robotisable selon la revendication 1 ou 2, dans lequel le système de transmission de mouvement (35) comprend :
- un premier pignon (36a) coaxial avec le premier organe d'entraînement (28a) et formant un élément d'entrée du système de transmission de mouvement (35),
- un pignon intermédiaire (37), présentant un axe (38) de pignon intermédiaire parallèle au premier axe (29a) et décalé par rapport à celui-ci, le pignon intermédiaire (37) étant en prise avec le premier pignon (36a) au moins dans toute configuration comprise entre les première et deuxième configurations,
- une transmission (39) entre le pignon intermédiaire (37) et le deuxième organe d'entraînement (29b), transmettant le mouvement de rotation du pignon intermédiaire (37) autour de l'axe (38) de pignon intermédiaire en ledit mouvement de rotation du deuxième organe d'entraînement (28b) autour du deuxième axe (29b).

4. Module robotisable selon la revendication 3 dans lequel le système élastique (46) sollicite le système d'actionnement (43) solidaire du deuxième organe d'entrainement (28b).

5. Module robotisable selon l'une des revendications 1 à 4, comprenant un système de verrouillage adapté pour alternativement verrouiller le deuxième organe d'entraînement (28b) dans sa configuration libre ou l'en libérer, le système d'actionnement (43) étant adapté pour commander le système de verrouillage.

6. Module robotisable selon l'une des revendications 1 à 5 dans lequel le système d'actionnement (43) est actionnable électriquement par l'utilisateur.

7. Module robotisable selon l'une des revendications 1 à 6 dans lequel au moins un organe d'entraînement (28a) est également monté mobile par rapport à la base (31) selon un mouvement de translation selon son axe (29a).

8. Module robotisable selon la revendication 7 dans lequel ledit au moins un organe d'entraînement (28a) est monté mobile par rapport à la base (31) selon un mouvement de translation selon son axe (29a) sur une course de translation,
Et dans lequel le premier organe d'entraînement (28a) comporte une jupe (81) déformable, frottant sur la base (31) lors de la rotation du premier organe d'entraînement (28a) par rapport à la base (31), et définissant un pourtour fermé sur la base (31) sur l'intégralité de la course de translation.

9. Module robotisable selon l'une quelconque des revendications 1 à 8 comprenant en outre un capot (32) solidaire de la base (31) et définissant avec celle-ci un boîtier (16) définissant un espace intérieur (41) dans lequel sont disposés au moins une partie du premier organe d'entraînement (28a), au moins une partie du deuxième organe d'entraînement (28b) et au moins une partie du système d'actionnement (43), et dans lequel une portion d'actionnement (44a) du système d'actionnement (43), une partie du premier organe d'entraînement (28a) et une partie du deuxième organe d'entraînement (28b) dépassent hors du boîtier (16).

10. Robot médical en kit comprenant une partie à demeure (51) et une partie amovible, la partie à demeure comprenant un moteur (34) et un premier accouplement (68, 68'), la partie amovible comprenant un module robotisable selon l'une des revendications 1 à 9 muni d'un deuxième accouplement (69, 69') complémentaire du premier accouplement (68, 68'),
dans lequel les premier et deuxième accouplements (68, 68', 69, 69') comprennent au moins une surface de came adaptée pour faire tourner l'un par rapport à l'autre le premier et le deuxième accouplements (68, 68', 69, 69') par rapport à une direction d'assemblage lors d'un assemblage de la partie amovible à la partie à demeure (51) selon la direction d'assemblage.

11. Robot médical en kit selon la revendication 10 dans lequel le premier accouplement (68, 68') comprend une pluralité de saillies (65, 62) de forme concave, et le deuxième accouplement (69, 69') comprend une pluralité complémentaire de logements (66, 64) de forme complémentaire.

12. Robot médical en kit selon la revendication 10 ou 11, dans lequel le premier accouplement comprend un cône de centrage (61), une saillie (62) mobile par rapport au cône de centrage (61) selon une direction de coulissement, et un organe de sollicitation (95) sollicitant la saillie (62) par rapport au cône de centrage (61) lors d'un assemblage de la partie amovible à la partie à demeure.

13. Système médical comprenant un organe médical souple creux allongé selon un axe d'élongation, et un robot médical selon l'une des revendications 10 à 12 ou un module robotisable selon les revendications 1 à 9, l'organe médical souple creux allongé (15) étant pris entre les première et deuxième surfaces périphériques d'entraînement (30a, 30b) dans la première configuration, le premier organe d'entraînement (28a) étant entraînable en rotation par rapport à la base (31) autour du premier axe (29a) pour générer une translation de l'organe médical souple allongé (15) selon son axe d'élongation.

14. Système médical selon la revendication 13, le premier organe d'entraînement (28a) étant entraînable en translation par rapport à la base (31) selon le premier axe (29a) pour générer une rotation de l'organe médical souple allongé (15) autour de son axe d'élongation.

## Patentansprüche

1. Robotisierbares Modul zum Antrieb eines langgestreckten flexiblen medizinischen Elements, umfassend:
- eine Basis (31),
- ein erstes Antriebselement (28a), das eine erste Achse (29a) definiert und eine erste periphere Antriebsfläche (30a) um diese erste Achse (29a) umfasst, wobei das erste Antriebselement (28a) in Bezug auf die Basis (31) um die erste Achse (29a) drehbar angebracht ist und ein Verbindungselement (33a) zu einem Antriebsmotor (34) umfasst, der dazu ausgebildet ist, das erste Antriebselement (28a) zur Drehung um die erste Achse (29a) anzutreiben,
- ein zweites Antriebselement (28b), das eine zweite Achse (29b) parallel zur ersten Achse (29a) definiert und eine zweite periphere Antriebsfläche (30b) um diese zweite Achse (29b) herum umfasst, wobei das zweite Antriebselement (29b) in Bezug auf die Basis (31) um die zweite Achse (29b) herum drehbar angebracht ist,
wobei das zweite Antriebselement (28b) in Bezug auf das erste Antriebselement (28a) gemäß einem anderen Freiheitsgrad als einer Drehung um die zweite Achse (29b) auch beweglich angebracht ist, zwischen:
- einer ersten Konfiguration, in der die erste und die zweite periphere Antriebsfläche (30a, 30b) einander mit einem ersten Abstand gegenüberliegen, und
- einer zweiten Konfiguration, in der die erste und die zweite periphere Antriebsfläche (30a, 30b) einander mit einem zweiten Abstand gegenüberliegen, der größer als der erste Abstand ist,
- ein von einem Benutzer betätigbares Betätigungssystem (43), das dazu ausgebildet ist, das zweite Antriebselement (28b) wenigstens von einer der ersten und zweiten Konfiguration in die andere der ersten und zweiten Konfiguration zu bewegen,
**gekennzeichnet durch** ein Bewegungsübertragungssystem (35) zum Übertragen der von dem Antriebsmotor (34) erzeugten Antriebsbewegung auf das zweite Antriebselement (28b), um das zweite Antriebselement (28b) wenigstens in jeder Konfiguration zwischen der ersten und der zweiten Konfiguration um die zweite Achse (29b) zu drehen,
und umfassend ein elastisches System (46), welches das zweite Antriebselement (28b) aus seiner zweiten Konfiguration in seine erste Konfiguration vorspannt,
und wobei das Betätigungssystem (43) betätigt werden kann, um das zweite Antriebselement (28b) von der ersten Konfiguration in die zweite Konfiguration zu bewegen, indem das elastische System (46) komprimiert wird.

2. Robotisierbares Modul nach Anspruch 1, wobei das Bewegungsübertragungssystem (35) in der freien Konfiguration, die die zweite Konfiguration ist, betrieben wird.

3. Robotisierbares Modul nach Anspruch 1 oder 2, wobei das Bewegungsübertragungssystem (35) umfasst:
- ein erstes Zahnrad (36a), das koaxial zu dem ersten Antriebselement (28a) ist und ein Eingangselement des Bewegungsübertragungssystems (35) bildet,
- ein Zwischenzahnrad (37), welches eine Zwischenzahnradachse (38) parallel zu und versetzt von der ersten Achse (29a) aufweist, wobei das Zwischenzahnrad (37) wenigstens in jeder Konfiguration zwischen der ersten und der zweiten Konfiguration mit dem ersten Zahnrad (36a) in Eingriff steht,
- eine Übertragung (39) zwischen dem Zwischenzahnrad (37) und dem zweiten Antriebselement (29b), welches die Drehbewegung des Zwischenzahnrads (37) um die Zwischenzahnradachse (38) in die genannte Drehbewegung des zweiten Antriebselements (28b) um die zweite Achse (29b) überträgt.

4. Robotisierbares Modul nach Anspruch 3, wobei das elastische System (46) das fest mit dem zweiten integralen Antriebselements (28b) verbundene Betätigungssystem (43) vorspannt.

5. Robotisierbares Modul nach einem der Ansprüche 1 bis 4, umfassend ein Verriegelungssystem, das dazu ausgebildet ist, das zweite Antriebselement (28b) alternativ in seiner freien Konfiguration zu verriegeln oder daraus zu lösen, wobei das Betätigungssystem (43) dazu ausgebildet ist, das Verriegelungssystem zu steuern.

6. Robotisierbares Modul nach einem der Ansprüche 1 bis 5, wobei das Betätigungssystem (43) durch den Benutzer elektrisch betätigbar ist.

7. Robotisierbares Modul nach einem der Ansprüche 1 bis 6, wobei wenigstens ein Antriebselement (28a) in Bezug auf die Basis (31) gemäß einer Translationsbewegung gemäß seiner Achse (29a) ebenfalls beweglich angebracht ist.

8. Robotisierbares Modul nach Anspruch 7, wobei das wenigstens eine Antriebselement (28a) in Bezug auf die Basis (31) gemäß einer Translationsbewegung entlang seiner Achse (29a) auf einem Translationsweg beweglich angebracht ist,
und wobei das erste Antriebselement (28a) eine verformbare Schürze (81) umfasst, die bei der Drehung des ersten Antriebselements (28a) in Bezug auf die Basis (31) an der Basis (31) reibt und über den gesamten Translationsweg einen festen Umfang an der Basis (31) definiert.

9. Robotisierbares Modul nach einem der Ansprüche 1 bis 8, ferner umfassend eine Haube (32), die fest mit der Basis (31) verbunden ist und mit dieser ein Gehäuse (16) begrenzt, das einen Innenraum (41) begrenzt, in dem wenigstens ein Teil des ersten Antriebselements (28a), wenigstens ein Teil des zweiten Antriebselement (28b) und wenigstens ein Teil des Betätigungssystems (43) angeordnet sind, und wobei ein Betätigungsabschnitt (44a) des Betätigungssystems (43), ein Teil des ersten Antriebselements (28a) und ein Teil des zweiten Antriebselements (28b) aus der Basis (31) des Gehäuses (16) herausragen.

10. Medizinischer Roboter als Bausatz, umfassend einen festen Teil (51) und einen lösbaren Teil, wobei der feste Teil einen Motor (34) und eine erste Kopplung (68, 68') umfasst, und der lösbare Teil ein robotisierbares Modul nach einem der Ansprüche 1 bis 9 umfasst, das mit einer zweiten Kopplung (69, 69') versehen ist, die komplementär zur ersten Kopplung (68, 68') ist,
wobei die erste und die zweite Kopplung (68, 68', 69, 69') wenigstens eine Nockenfläche umfassen, die dazu ausgebildet ist, die erste und die zweite Kopplung (68, 68', 69, 69') relativ zueinander in Bezug auf eine Montagerichtung zu drehen, wenn der lösbare Teil mit dem festen Teil (51) gemäß der Montagerichtung zusammengebaut wird.

11. Medizinischer Roboter als Bausatz nach Anspruch 10, wobei die erste Kopplung (68, 68') eine Vielzahl von konkav geformten Vorsprüngen (65, 62) und die zweite Kopplung (69, 69') eine komplementäre Vielzahl von komplementär geformten Aufnahmen (66, 64) umfasst.

12. Medizinischer Roboter als Bausatz nach Anspruch 10 oder 11, wobei die erste Kopplung einen Zentrierkegel (61), einen Vorsprung (62), der in Bezug auf den Zentrierkegel (61) in einer Gleitrichtung beweglich ist, und ein Vorspannelement (95) umfasst, das den Vorsprung (62) in Bezug auf den Zentrierkegel (61) beim Zusammenbau des lösbaren Teils mit dem festen Teil vorspannt.

13. Medizinisches System, umfassend ein hohles, flexibles, medizinisches Element, das gemäß einer Verlängerungsachse langgestreckt ist, und einen medizinischen Roboter nach einem der Ansprüche 10 bis 12 oder ein robotisierbares Modul nach den Ansprüchen 1 bis 9, wobei das hohle, flexible, medizinische Element (15) in der ersten Konfiguration zwischen einer ersten und einer zweiten peripheren Antriebsfläche (30a, 30b) aufgenommen ist, wobei das erste Antriebselement (28a) in Bezug auf die Basis (31) um die erste Achse (29a) drehbar ist, um eine Translation des langgestreckten flexiblen medizinischen Elements (15) um seine Verlängerungsachse herum zu erzeugen.

14. Medizinisches System nach Anspruch 13, wobei das erste Antriebselement (28a) in Bezug auf die Basis (31) gemäß der ersten Achse (29a) translatorisch angetrieben werden kann, um eine Drehung des langgestreckten flexiblen medizinischen Elements (15) um seine Verlängerungsachse zu erzeugen.

## Claims

1. Robotisable module for driving an elongated flexible medical member, comprising:
- a base (31),
- a first drive member (28a) defining a first axis (29a), and comprising a first peripheral drive surface (30a) around this first axis (29a), the first drive member (28a) being mounted mobile in rotation relative to the base (31) about the first axis (29a), and comprising a member (33a) for linking to a drive motor (34) adapted to drive the first drive member (28a) in rotation about the first axis (29a),
- a second drive member (28b) defining a second axis (29b) parallel to the first axis (29a), and comprising a second peripheral drive surface (30b) around this second axis (29b), the second drive member (29b) being mounted mobile in rotation relative to the base (31) about the second axis (29b),
the second drive member (28b) also being mounted so as to be mobile relative to the first drive member (28a) according to a degree of freedom other than rotational about the second axis (29b), between:
- a first configuration in which the first and second peripheral drive surfaces (30a, 30b) face each other with a first spacing, and
- a second configuration in which the first and second peripheral drive surfaces (30a, 30b) face each other with a second spacing greater than the first spacing,
- an actuation system (43) actuatable by a user, adapted to make the second drive member (28b) go at least from one of the first and second configurations to the other of the first and second configurations,
**characterised by** a motion transmission system (35) for transmitting the drive movement generated by the drive motor (34) to the second drive member (28b) to drive the second drive member (28b) in rotation about the second axis (29b) at least in any configuration between the first and second configurations
and comprising an elastic system (46) stressing the second drive member (28b) from its second configuration towards its first configuration,
and wherein the actuation system (43) is actuatable to make the second drive member (28b) go from the first configuration to the second configuration while compressing said elastic system (46).

2. Robotisable module according to claim 1, wherein the motion transmission system (35) is operative in the free configuration which is the second configuration.

3. Robotisable module according to claim 1 or 2, wherein the motion transmission system (35) comprises:
- a first gear (36a) coaxial to the first drive member (28a) and forming an input element of the motion transmission system (35),
- an intermediate gear (37), having an intermediate gear axis (38) parallel to the first axis (29a) and offset relative to the latter, the intermediate gear (37) meshing with the first gear (36a) at least in any configuration between the first and second configurations,
- a transmission (39) between the intermediate gear (37) and the second drive member (29b), transmitting the rotational motion of the intermediate gear (37) about the intermediate gear axis (38) into said rotational motion of the second drive member (28b) about the second axis (29b).

4. Robotisable module according to claim 3, wherein the elastic system (46) stresses the actuation system (43) integral with the second drive member (28b).

5. Robotisable module according to one of claims 1 to 4, comprising a locking system adapted to alternatingly lock the second drive member (28b) in its free configuration or release it therefrom, the actuation system (43) being adapted to control the locking system.

6. Robotisable module according to one of claims 1 to 5, wherein the actuation system (43) is electrically actuatable by the user.

7. Robotisable module according to one of claims 1 to 6, wherein at least one drive member (28a) is also mounted mobile relative to the base (31) according to a motion of translational along its axis (29a).

8. Robotisable module according to claim 7, wherein said at least one drive member (28a) is mounted mobile relative to the base (31) according to a motion of translation along its axis (29a) on a translation path,
and wherein the first drive member (28a) includes a deformable skirt (81), rubbing on the base (31) during the rotation of the first drive member (28a) relative to the base (31), and defining a closed perimeter on the base (31) over the entirety of the translation path.

9. Robotisable module according to any one of claims 1 to 8, further comprising a cover (32) rigidly connected to the base (31) and defining with the latter a case (16) defining an inner space (41) in which at least a part of the first drive member (28a), at least a part of the second drive member (28b) and at least a part of the actuation system (43) are disposed, and wherein an actuation portion (44a) of the actuation system (43), a part of the first drive member (28a) and a part of the second drive member (28b) protrude out of the housing (16).

10. Medical robot in a kit, comprising a permanent part (51) and a removable part, the permanent part comprising a motor (34) and a first coupling (68, 68'), the removable part comprising a robotisable module according to one of claims 1 to 9 provided with a second coupling (69, 69') complementary to the first coupling (68, 68'),
wherein the first and second couplings (68, 68', 69, 69') comprise at least one cam surface adapted to make the first and the second couplings (68, 68', 69, 69') rotate relative to one another with respect to a direction of assembly during an assembly of the removable part to the permanent part (51) according to the direction of assembly.

11. Medical robot in a kit according to claim 10, wherein the first coupling (68, 68') comprises a plurality of protrusions (65, 62) having a concave shape, and the second coupling (69, 69') comprises a complementary plurality of housings (66, 64) having a complementary shape.

12. Medical robot in a kit according to claim 10 or 11, wherein the first coupling comprises a centring cone (61), a protrusion (62) mobile relative to the centring cone (61) in a sliding direction, and a stressing member (95) stressing the protrusion (62) relative to the centring cone (61) during an assembly of the removable part to the permanent part.

13. Medical system comprising a hollow flexible medical member elongated along an axis of elongation, and a medical robot according to one of claims 10 to 12 or a robotisable module according to claims 1 to 9, the elongated hollow flexible medical member (15) being held between the first and second peripheral drive surfaces (30a, 30b) in the first configuration, the first drive member (28a) being drivable in rotation relative to the base (31) about the first axis (29a) to generate a translation of the elongated flexible medical member (15) along its axis of elongation.

14. Medical system according to claim 13, the first drive member (28a) being drivable in translation relative to the base (31) along the first axis (29a) to generate a rotation of the elongated flexible medical member (15) about its axis of elongation.
